# EUROPÄISCHE PATENTSCHRIFT

(11) **EP 0 485 881 B1**
(45) Veröffentlichungstag und Bekanntmachung des Hinweises auf die Patenterteilung: **18.12.1996**
(21) Anmeldenummer: 91118914.0
(22) Anmeldetag: 06.11.1991
(51) Int. Cl.: D01G 31/00, G01N 33/36, G01N 21/89, D01G 23/06, B65H 63/06

(54) **Verfahren und Vorrichtung zur Steuerung einer Karde**
Method and device for controlling a card
Procédé et dispositif pour commander une carde

(30) Priorität: 14.11.1990 CH 3615/90
(43) Veröffentlichungstag der Anmeldung: 20.05.1992
(73) Patentinhaber: MASCHINENFABRIK RIETER AG, CH-8406 Winterthur (CH)
(72) Erfinder: Demuth, Robert, CH-8309 Nürensdorf (CH); Fritzsche, Peter, CH-8405 Winterthur (CH)

(56) Entgegenhaltungen:
- DE-A- 1 648 430
- GB-A- 2 170 316
- GB-A- 2 210 907
- US-A- 3 157 915

## Beschreibung

Die Erfindung liegt auf dem Gebiete der Textilfaserverarbeitung und betrifft ein Verfahren und eine Vorrichtung, um in einer Karde mit einem minimalen Mess- und Verarbeitungsaufwand Messwerte zu gewinnen, die mit der Kardierungsqualität, insbesondere mit der die Kardierungsqualität stark bestimmenden Faserbeeinträchtigung, korreliert werden können und mit deren Hilfe die Kardenparameter derart eingestellt und gesteuert oder reguliert werden können, dass die Karde aus einer möglichst weiten Palette von Faserprovenienzen optimal kardierte Produkte herstellen kann, das heisst Kardenprodukte, die für eine auf die Karde folgende, bestimmte Weiterverarbeitung genügend gereinigt und parallelisiert, deren Fasern aber nur in einem tolerierbaren Mass durch die Kardierung- beeinträchtigt sind.

DE 1648430 setzt sich zum Ziel, ein Verfahren zu schaffen, mittels dessen ein Faserverband auf Trash bzw. Nissen geprüft werden kann, ohne dass durch das andere Parameter das Messergebnis verfälscht wird. Dazu wird der Faserverband mittels einer Strahlung beaufschlagt und sowohl die vom Faserverband reflektierte als auch die durchgehende Strahlung gefühlt. Das Verfahren macht sich die Tatsache zunutze, dass Trash und Nissen deutlich unterschiedliche Reflektionskoeffizienten aufweisen, wobei bestimmte Wellenlängenbereiche besonders grosse Unterschiede der erwähnten Reflektionskoeffizienten ergeben.

Fasern werden in der Karde zwischen stationären und rotierenden Garnituren parallelisiert und gereinigt, wobei Verschmutzungspartikel ausgeschieden werden. Je nach Provenienz, Verschmutzungs- und Parallelitätsgrad des in die Karde eingespeisten Fasermaterials und je nach den Anforderungen an das kardierte Produkt muss der Kardierprozess verschieden geführt werden. Die übliche Art, Karden an verschiedene Anforderungen anzupassen, besteht darin, diese ausser Betrieb zu demontieren und die stationären Garnituren auszuwechseln und/oder deren Abstand zu den rotierenden Garnituren einzustellen. Es sind aber in neuester Zeit Anstrengungen gemacht worden, um solche Einstellarbeiten zu minimieren und Kardierelemente zu entwickeln, die möglichst von aussen und möglichst auch während dem Betrieb der Karde verstellt werden können. So beschreibt zum Beispiel die Europäische Patentanmeldung Nr. 0403989 derselben Anmelderin Kardierelemente, mit deren Hilfe die meisten Kardenparameter von ausserhalb der Maschine und während dem Betrieb eingestellt werden können. Solche Kardenparameter sind beispielsweise: die Garniturarten (Zähne, Nadeln etc), die Feinheit der Garnituren, der Anstellwinkel der Garniturelemente, der Abstand zwischen stationären und rotierenden Garnituren, der Verlauf dieses Abstandes in Kardierrichtung und die Drehzahl der Tambourwalze.

Mit Hilfe dieser Kardenparameter kann nun eine Karde sehr genau den jeweiligen Anforderungen angepasst werden. Zu diesem Zwecke sind aber viele Vorversuche und vor allem nach jedem Wechsel der Faserprovenienz oder der Anforderungen an das kardierte Produkt entsprechende Anlaufphasen notwendig, während denen sehr erfahrenes Personal die Karde beaufsichtigen muss oder das von der Karde gelieferte Produkt immer wieder im Labor untersucht werden muss. Auch die Überwachung der Arbeitsqualität und der Funktionstüchtigkeit der Karde kann nur durch periodische Analysen des Kardenproduktes bewerkstelligt werden. Alle diese Methoden sind aufwendig und zeitraubend und machen zu wenig automatisierenden Gebrauch von den Einstellmöglichkeiten der Kardiermaschinen der neuesten Generationen.

Für eine einfachere und sicherere Anlaufphase der Karde, für eine automatisierte Steuerung und Regulierung der Kardenparameter und Überwachung der Karde, sowie ganz allgemein dafür, dass die Qualität des kardierten Produktes schnell und auch von weniger erfahrenem Personal eingeschätzt werden kann, wäre ein System vorteilhaft, mit dem on-line wichtige Qualitätsmerkmale des Kardenproduktes gemessen werden können.

Ein solches Qualitätsmerkmal des kardierten Produktes ist beispielsweise die Anzahl der in diesem Produkt vorhandenen Nissen. Eine Vorrichtung zum On-line-Detektieren und Zählen von Nissen in einer Karde wird zum Beispiel beschrieben in der Schweizer Patentschrift 669 401. Es wird darin vorgeschlagen, das Kardenvlies, bevor es zu einem Band zusammengefasst wird, also beispielsweise auf der Abnehmerwalze, entsprechend zu beleuchten und mit über die Breite des Vlieses verteilten, optischen Sensoren zu überwachen. Nissen zeigen sich als Punkte erhöhter Reflektion und können durch eine elektronische Auswertschaltung einfach (Signalverlauf über einem bestimmten Pegel) vom übrigen Signalverlauf getrennt und gezählt werden.

Ein weiteres sehr wichtiges Qualitätsmerkmal für das Kardenprodukt ist die Faserbeeinträchtigung. Die Faserbeeinträchtigung ist ein Mass für die Schädigung der Fasern bei der Kardierung. Diese Schädigung nimmt mit steigender Kardier-Intensität zu, das heisst die Qualität des kardierten Produktes bezüglich Faserbeeinträchtigung sinkt mit steigender Kardier-Intensität, während die Qualität beispielsweise bezüglich Verschmutzung oder Nissengehalt steigt. Um also die Kardierqualität ganzheitlich on-line kontrollieren zu können, ist es unumgänglich, auch die Faserbeeinträchtigung oder mindestens Veränderungen in der Faserbeeinträchtigung on-line feststellen zu können.

Die Erfindung stellt sich nun die Aufgabe, ein Verfahren und eine Vorrichtung aufzuzeigen, mit dem Veränderungen in der Faserbeeinträchtigung des kardierten Produktes oder die Faserbeeinträchtigung selbst on-line ermittelt werden können. Im einfachsten Falle sollen Messwerte, die der Faserbeeinträchtigung oder einer Veränderungen in der Faserbeeinträchtigung korrelierbar sind, angezeigt und/oder protokolliert werden, damit der Maschinen-Operator sie mit empirisch gewonnenen Vergleichswerten (Eichwerten) vergleichen und entsprechende Massnahmen einleiten kann. Es soll auch möglich sein, die Messwerte automatisch mit entsprechenden Eichwerten zu vergleichen, sodass direkt die Faserbeeinträchtigung oder deren Veränderung angezeigt und oder protokolliert werden kann. Dabei sollen vor allem auch sehr langsame Veränderungen angezeigt werden können. Die gemessenen Grössen sollen auch dazu dienen, automatische Alarme beim Überschreiten von Grenzwerten auszulösen, und sie sollen als Messgrössen für automatische Steuerung oder Regulierung der Kardenparameter verwendet werden. Die ermittelten Messwerte sollen es also beispielsweise ermöglichen, die kontinuierliche Abnutzung der Kardengarnituren zu verfolgen, die Kardeneinstellung anhand der Produktqualität zu überprüfen, die Karde nach dem Anlaufen fein einzustellen und bei laufender Maschine auf eine gewünschte, optimale Qualität des kardierten Produktes zu regulieren und Fehlfunktionen sofort festzustellen. Das Verfahren soll mit möglichst wenig messtechnischem und elektronischem Aufwand durchführbar sein.

Ein nicht zu unterschätzendes Problem ist die Messung langsamer Vorgänge, wie trendartige Veränderungen an schnellbewegten Objekten, wie das sich vorbeibewegende Vlies. Beides zeitabhängige Vorgänge, jedoch in völlig verschiedenen Grössenordnungen, derart verschieden, dass im Bereich der Vliesgeschwindigkeit die trendartige Änderung als quasi stationär (zeitunabhängig) erscheint.

Die gestellte Aufgabe wird gelöst durch das in den Patentansprüchen angegebene Verfahren. Ein Beispiel wird im folgenden anhand von Figuren erläutert. Dabei zeigen:
- **Figur 1**: ein beispielhaftes Verfahrensschema,
- **Figur 2**: ein Diagramm zur Erläuterung der Funktion des Analysators,
- **Figur 3**: den Zusammenhang zwischen der Kardierungsintensität und den Qualitätsmerkmalen Faserbeeinträchtigung, Verschmutzung und Nissenzahl.
- **Figur 4**: ein Vorrichtungsvorschlag zur Durchführung des Verfahrens.

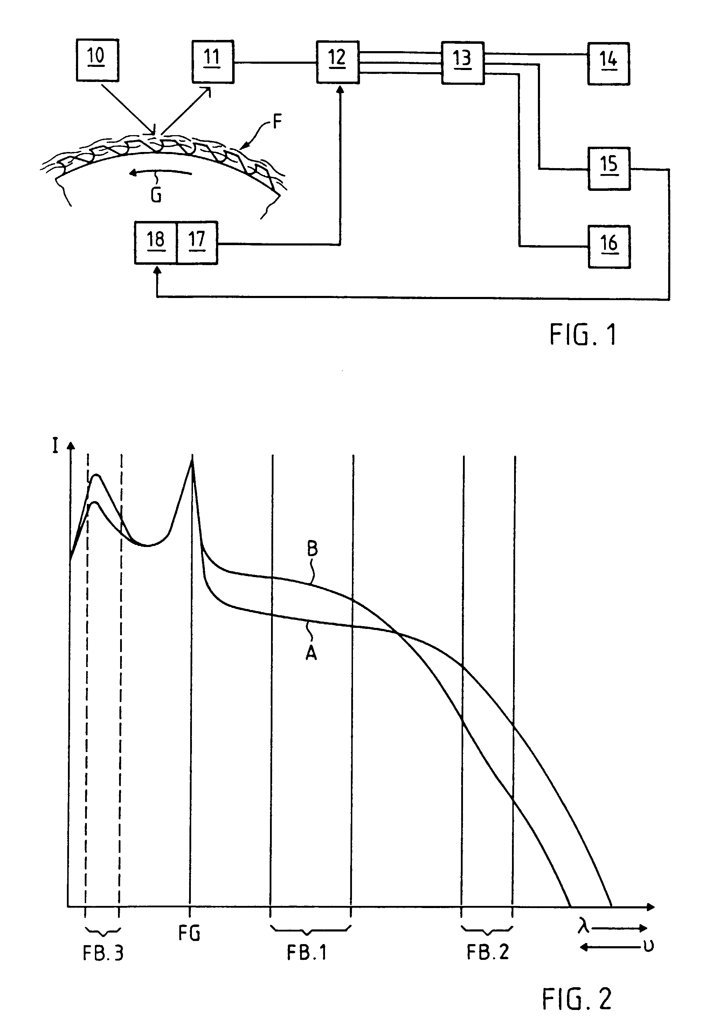

**Figur 1** zeigt ein Schema für eine beispielhafte Variante des erfindungsgemässen Verfahrens. Ein genügend dünnes Faservlies F bewegt sich kontinuierlich mit der Geschwindigkeit G, zum Beispiel das Fasermaterial auf dem Tambour einer Karde mit der Oberflächengeschwindigkeit des Tambours. Das Fasermaterial wird mit Gleichlicht (zeitunabhängiges Licht) aus mindestens einer Gleichlichtquelle 10 beleuchtet. Das Gleichlicht wird vom Fasermaterial und eventuell auch von das Fasermaterial transportierenden Garnituren moduliert und reflektiert. Das reflektierte, modulierte Licht (zeitabhängiges Licht), das sehr viel Information über das Fasermaterial enthält, wird von mindestens einem optischen Helligkeitssensor 11 aufgenommen und in eine modulierte elektrische Spannung verwandelt. Da der Sensor 11 die ganze Menge des von ihm empfangenen Lichtes zu einem zeitabhängigen Signal verarbeitet, das einem Mittelwert des von ihm in jedem Zeitpunkt erblickten Bildes entspricht, ist es notwendig, dass die Grösse des Sensors der Grösse der interessierenden Gegenstände etwa entspricht, damit dieser Mittelwert noch aussagekräftig ist. Es hat sich gezeigt, dass ein solcher Sensor vorzugsweise in der Bewegungsrichtung des Fasermaterials eine Abmessung von ca. 0,5mm, quer zur Bewegungsrichtung des Fasermaterials eine Abmessung von ca 2mm besitzt. Das heisst mit anderen Worten dass der Sensor, verglichen mit der Breite des Vlieses, sehr klein ist und dass es sinnvoll ist mehrere solcher Sensoren über diese Breite anzuordnen, deren Signale dann beispielsweise über einen Multiplexer nacheinander weitergeleitet werden können.

Der Einfallswinkel des Gleichlichtes auf das Fasermaterial und die relative Position von Lichtquelle 10 und Sensor (oder Sensoren) 11 müssen durch Versuche für eine optimale Funktion des Verfahrens eingestellt werden.

Das resultierende, komplexe Signal (zeitabhängiges Messsignal) des Sensors (oder der Sensoren) 11 wird in einen Analysator 12 geleitet, der aus dem Gesamt-Frequenzbereich des Messsignales bestimmte Bereiche ausfiltert und deren Intensitäten misst. Eine beispielhafte Funktionsweise für einen solchen Analysator wird durch die **Figur 2** illustriert, die im folgenden noch detailliert beschrieben werden soll.

Die Ausgangssignale (zeitabhängige Ausgangswerte) der Frequenzanalyse werden einer Verarbeitungseinheit 13 zugeführt. Die Verarbeitungseinheit 13 hat beispielsweise die folgenden Aufgaben:
- Bildung sinnvoller zeitlicher Mittelwerte, die für die Beobachtung von einerseits langwelligen und andererseits kurzwelligen Veränderungen verwendet werden können,
- Bildung sinnvoller örtlicher Mittelwerte, die beispielsweise die Information der über die Breite des Kardenvlieses verteilten Sensoren zusammenfassen,
- Bildung von Werten, die mit der Faserqualität, insbesondere mit der Faserbeeinträchtigung, korreliert werden können, falls die Ausgangswerte der Frequenzanalyse oder entsprechende Mittelwerte nicht direkt weiterverwendet werden können (detaillierte Beschreibung von Beispielen im Zusammenhang mit der Figur 2),
- Vergleich der ermittelten Werte mit empirisch ermittelten, gespeicherten Eichwerten zur Ermittlung von Werten der effektiven Faserbeeinträchtigung,
- Vergleich der ermittelten Werte mit gespeicherten Grenzwerten zur Erzeugung von Alarmsignalen.

Aus den aufgelisteten Aufgaben der Verarbeitungseinheit 13 geht hervor, dass die Verarbeitungseinheit mit einem entsprechenden Speicher ausgestattet sein muss, in dem die ermittelten Werte zur Bildung von Kurzzeit- und Langzeit-Mittelwerten und empirisch ermittelte Vergleichswerte gespeichert werden.

Ausgewählte, durch die Verarbeitungseinheit 13 aus den Ausgangssignalen des Analysators 12 ermittelte Werte werden auf Ausgabeeinheiten 14 geführt. Diese können LCD-Anzeigen direkt an der Maschine oder Bildschirme zur simultanen Überwachung sein oder Drucker zur Erstellung von Protokollen. Die von der Verarbeitungseinheit 13 ermittelten Werte können auch einer Regeleinheit 15 zugeführt werden, die sie zu Stellgrössen für die einzelnen Kardenparameter verarbeitet und an entsprechende Stellwerke 18 für die Verstellung der Kardenparameter weiterleitet. Die von der Verarbeitungseinheit 13 erzeugten Alarmsignale werden auf Alarmeinheiten 16 geleitet, die dadurch entsprechend aktiviert werden.

**Figur 2** zeigt im Detail eine beispielhafte Funktionsweise der Frequenzanalyse, die der Analysator 12 an einem von einem optischen Sensor 11 gelieferten Signal ausführt. Auf der Abszisse des Diagrammes ist die Frequenz ν gegen rechts abnehmend, oder die Wellenlänge λ gegen rechts zunehmend, auf der Ordinate die Intensität I aufgetragen. Die beiden Kurven A und B stellen zum Beispiel zwei nacheinander aufgenommene Frequenzspektren des vom optischen Sensor 11 gelieferten Signales dar. Es kann sich dabei um "Momentaufnahmen" handeln oder um Mittel über einen kürzeren oder längeren Zeitraum. Es ist nun die Aufgabe des Analysators 12, aus diesem Frequenzspektrum Grössen herauszufiltern, die mit der Faserbeeinträchtigung korreliert werden können.

Die Faserbeeinträchtigung wirkt sich unter anderem durch Faserrisse aus, das heisst, wenn ein Fasermaterial beeinträchtigt wird, sind nachher ein Teil der Fasern gerissen, also kürzer als vorher. Die Faserbeeinträchtigung kann also bestimmt werden, indem die mittlere Stapellänge bestimmt wird oder aber indem der relative Anteil an kürzeren Fasern oder das Verhältnis der Anteile von längeren zu kürzeren Fasern ermittelt wird und indem diese Werte mit entsprechenden Werten des Ausgangsproduktes verglichen werden. Sind die Werte für Ausgangsprodukt und Kardierungsprodukt gleich, ist das Fasermaterial durch die Kardierung nicht beeinträchtigt worden, sind die Werte verschieden (der Anteil an kürzeren Fasern höher), so ist das Fasermaterial mehr oder weniger stark beeinträchtigt worden. Aus der Erfahrung sind tolerierbare Werte einer solchen Faserbeeinträchtigung bekannt, die je nach Faserprovenienz und je nach der auf das Kardieren folgende Weiterverarbeitungsart verschieden sein können.

Es zeigt sich nun, dass aus dem Frequenzspektrum gemäss Figur 2, das das von einem optischen Sensor aufgenommene, von einem Kardenvlies reflektierte Licht repräsentiert, Werte ausgefiltert werden können, die mit der Faserlänge, also schliesslich auch mit der Faserbeeinträchtigung korrelierbar sind.

Die Form der in der Figur 2 dargestellten Kurve wird sicher bestimmt durch die Garnitur, die mit einer mehr oder weniger konstanten Geschwindigkeit unter dem Sensor vorbeigeführt wird und deshalb bei einer bestimmten, durch die Garniturdichte und die Tambourgeschwindigkeit bestimmten Frequenz, beispielsweise FG in Figur 2, eine konstante Intensität ergibt. Auch die Fasern, die mehr oder weniger gestreckt wegen der geringen Sensorgrösse und der geringen Dichte des Fasermaterials in geringer Zahl unter dem Sensor durchlaufen, werden die Kurve beeinflussen und zwar bei Frequenzen, die grössenordnungsmässig der Faserlänge entsprechen, aber sicher auch noch durch die relative Wirrlage beeinflusst sind, und mit Intensitäten, die vom relativen Anteil von Fasern des entsprechenden Faserlängenbereiches bestimmt werden. Die Frequenzen, die die Fasern repräsentieren, sind niedriger als die Frequenz der Garnitur und in der Figur 2 also rechts von FG zu erwarten. Variationen in der durchschnittlichen Dichte an Fasern des beobachteten Fasermaterials, die durch mehr oder weniger aufgelöste Flocken entstehen können, werden zu Intensitäten bei noch niedrigeren Frequenzen (höheren Wellenlängen, noch weiter rechts auf dem Diagramm) führen. Wie die Frequenz der Garnitur sind auch alle anderen Frequenzen nicht nur abhängig von der Natur der sie bewirkenden Gegenstände sondern auch von der Geschwindigkeit G, mit der sich das Fasermaterial unter dem Sensor durchbewegt, also von der Umdrehungszahl des Tambours.

Der Analysator kann nun beispielsweise so ausgelegt sein, dass er 2 Frequenzbänder ausfiltert: ein erstes Frequenzband FB.1 über einen Frequenzbereich, der Fasern mit einer kleineren Faserlänge entspricht, und ein zweites Frequenzband FB.2 über einen Frequenzbereich, der Fasern mit einer grösseren Faserlänge entspricht. Die beiden Frequenzbänder liegen zwischen der Frequenz der Garnitur FG und dem den Flocken entsprechenden Frequenzbereich. Der Analysator misst nun beispielsweise die zeitabhängige, mittlere Intensität über jedem der beiden Frequenzbänder: I(FB.1) und I(FB.2). Diese beiden Werte sind direkt korrelierbar mit den Anteilen an kürzeren und längeren Fasern. Die Verarbeitungseinheit kann aus diesen beiden Werten beispielsweise einen Quotienten I(FB.1)/I(FB.2) bilden, wodurch ein Einfluss der Faserdichte auf die Intensitäten ausgeschaltet wird. Weicht der Wert des Quotienten von einem vorgegebenen, durch entsprechende Labormessungen bestimmten Wert für eine optimale Verarbeitung der zu verarbeitenden Faserprovenienz ab, kann daraus auf eine Abweichung der Kardierungsintensität von ihrer für das entsprechende Produkt optimalen Einstellung geschlossen werden, sofern eine Veränderung in der Qualität des Ausgangsproduktes ausgeschlossen werden kann. Für eine Konstanthaltung der Qualität des Kardierungsproduktes muss also in einem solchen Fall die Kardierungsintensität über die Kardenparameter entsprechend anders eingestellt werden.

Das Resultat der Frequenzanalyse ist in keinem Fall ein Wert für ein physikalisches Qualitätsmerkmal des Kardenproduktes, wie beispielsweise die Stapellänge oder deren Veränderung durch Faserbeeinträchtigung, aber die durch die Analyse ermittelten Werte sind mit effektiven Grössen von Qualitätsmerkmalen derart korreliert, dass aus Veränderungen der einen auf Veränderungen der anderen und von den ermittelten Werten mit Hilfe von empirisch ermittelten Eichgrössen schliesslich auf die Qualitätsmerkmale selbst geschlossen werden kann.

Die vorteilhafteste Lage der beiden Frequenzbänder FB.1 und FB.2 ist abhängig von der Karde und von der Art des verarbeiteten Fasermaterials. Sie muss empirisch durch Versuche ermittelt werden.

Ein entsprechender Analysator kann realisiert werden, indem das vom optischen Sensor erzeugte Signal auf zwei Bandpasssysteme geleitet wird, die je ein Frequenzband durchlassen (ausfiltern).

Wenn die Geschwindigkeit G des Fasermaterials nicht konstant ist, beispielsweise durch Regulierung der Tambourdrehzahl, muss die Lage der ausgefilterten Frequenzbänder entsprechend reguliert werden, damit die Analysenwerte vergleichbar werden. Dies kann, wie in der Figur 1 angedeutet, mit einer "äusseren" Regulierung des Bandpasssystemes, mit Hilfe eines Signales beispielsweise einer entsprechenden Messstelle 17 an der Karde realisiert werden. Es kann aber auch die konstante Intensität der Garniturfrequenz FG für diesen Zweck ausgenützt ("innere" Regulierung) werden, indem das Bandpasssystem entsprechend der Frequenzlage der in einem möglichen Frequenzbereich für die Garnitur gefundenen höchsten Intensität reguliert wird. Das heisst mit anderen Worten, dass die Intensitäten I(FB.1) und I(FB.2) in einem konstanten Frequenzabstand von der Frequenz des Intensitätspeaks, der der Garnitur zugeordnet werden kann, gemessen werden.

Statt zwei kann auch nur ein Frequenzband ausgefiltert werden. Bei gut konstanter Faserdichte auf dem Tambour kann die Bestimmung der Intensität eines Frequenzbandes, das einer hohen Faserlänge entspricht für die Bestimmung der Faserbeeinträchtigung genügen.

Statt die Intensitäten in den ausgefilterten Frequenzbändern zu messen, kann auch die Frequenzbegrenzung der Bänder derart verändert werden, dass die Intensität konstant bleibt. Wie oben beschrieben eine Änderung in der Intensität gibt dann eine Änderung in der Lage der Frequenzbegrenzung der Bänder eine Grösse, die mit einer Veränderung der relativen Anteile von Fasern bestimmter Faserlängenbereiche, also mit der Faserbeeinträchtigung korreliert werden kann. Die entsprechenden Intensitäten müssen ebenfalls empirisch ermittelt werden.

Statt einer einfachen Ausfilterung von Frequenzbändern kann das von dem optischen Sensor gelieferte Signal auch einer Fourier-Transformation unterzogen werden. Die aus der Fourier-Transformation gewonnene Information ist aber mit bedeutend mehr Aufwand verbunden und kann ebenfalls keine Werte liefern, die direkt Messwerte für die Faserbeeinträchtigung sind, sondern, wie das Verfahren mit den Frequenzbändern, nur Werte, die mit der Faserbeeinträchtigung über entsprechende Eichwerte korreliert werden können.

Für die Weiterverarbeitung der aus der Frequenzanalyse anfallenden zeitabhängigen Signale sind in jedem Falle Erfahrungswerte oder Vergleichswerte aus entsprechenden Labormessungen notwendig, unabhängig davon, ob die Weiterverarbeitung durch das Personal oder automatisch durchgeführt wird. Erfahrungswerte oder Vergleichswerte sind notwendig für das optimal kardierte Produkt aus einem bestimmten Ausgangsprodukt, für noch tolerierbare Abweichungen davon und für nicht mehr tolerierbare Grenzwerte. Sie sind ebenfalls notwendig, wenn die durch die Frequenzanalyse ermittelten Werte zu Werten des effektiven Qualitätsmerkmal Faserbeeinträchtigung verarbeitet werden sollen. Für automatische Vergleiche müssen entsprechende Eichwerte oder ganze Eichspektren in der Verarbeitungseinheit 13 gespeichert werden können.

Um Langzeitveränderungen festzustellen, werden bspw. aufeinanderfolgende Messungen miteinander verglichen. Da die Messungen zeitlich weit auseinanderliegen (Grössenordnung Stunden), können Mittelwerte von früheren Messabschnitten mit den Mittelwerten der aktuellen Messabschnitte miteinander verglichen werden. Durch den Vergleich aufeinanderfolgender Messungen, was mit relativ geringem apparativen Aufwand möglich ist, können trendartige Abweichungen festgestellt und protokolliert werden. Ein solches Protokoll dient dann zur Korrektur des Kardiervorganges.

Für eine ganzheitliche Beurteilung der Qualität des Kardenproduktes ist es vorteilhaft, nicht nur die Faserbeeinträchtigung, sondern auch andere Qualitätsparameter on-line zu kontrollieren. Wie bereits eingangs erwähnt, vermindert sich die Qualität bezüglich Faserbeinträchtigung mit steigender Kardierungsintensität, während die Qualität beispielsweise bezüglich Verschmutzung oder Nissen sich erhöht. Eine optimale Kardierung stellt also immer einen Kompromiss zwischen verschiedenen Qualitätsanforderungen dar. Wie dieser Kompromiss gewählt wird, hängt im weiteren von der Weiterverarbeitung des Kardenproduktes ab.

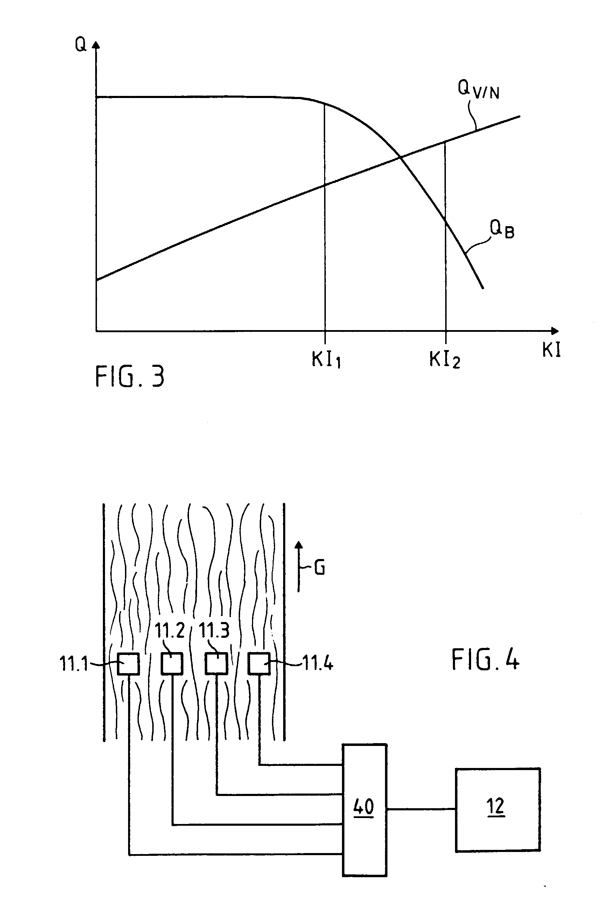

**Figur 3** zeigt ein entsprechendes Diagramm für die Qualität des Kardenproduktes (Ordinate Q) in Abhängigkeit der Kardierungsintensität (Abszisse KI), die durch die Einstellung der Kardenparameter bestimmt wird. Das Diagramm zeigt, dass bei steigender Intensität der Kardierung die Qualität des Produktes bezüglich Verschmutzung oder Nissenzahl (Q_{V/N}) sich erhöht, dass aber gleichzeitig die Qualität bezüglich Faserbeeinträchtigung (Q_{B}) sinkt, speziell über einer gewissen Kardierungsintensität. Mit steigender Kardierungsintensität KI werden zwar mehr Verschmutzungsanteile ausgeschieden und sinkt zwar die Nissenzahl, die Faserbeeinträchtigung aber, die sich durch eine Reduktion der mittleren Stapellänge auswirkt, steigt gleichzeitig an. Daraus ist sofort ersichtlich, dass kein eindeutiges Qualitätsoptimum besteht und dass die Qualität nur durch eine kombinierte Bestimmung von Faserbeeinträchtigung, Verschmutzung und Nissenzahl ganzheitlich beurteilt werden kann. Je nach weiterem Verarbeitungsprozess ist es wichtiger, dass das Kardenprodukt möglichst wenig Verschmutzungen und Nissen enthält, oder aber dass die Fasern möglichst ihre originale Stapellänge bewahren, also durch den Kardierungsprozess möglichst nicht beeinträchtigt werden. Das Qualitätsoptimum liegt also je nach weiterem Verarbeitungsverfahren da, wo die Faserbeeinträchtigung eine Toleranzgrenze nicht überschreitet und die Nissenzahl möglichst gering ist (z.B. KI₁ für Ringspinnerei), oder wo die Nissenzahl eine Toleranzgrenze nicht überschreitet und die Faserbeeinträchtigung möglichst gering ist (z.B. KI₂ für Open-End-Spinnerei). Für eine ganzheitliche Beurteilung der Qualität des Kardenproduktes, die zu einer optimalen Einstellung und Regulierung der Kardenparameter dienen kann, ist es also vorteilhaft, neben der On-line-Bestimmung der Faserbeeinträchtigung auch On-line-Messungen für Verschmutzung und/oder Nissenzahl einzusetzen und die damit gewonnene Information in der beschriebenen Weise einzusetzen. Für die Messung der Nissenzahl kann beispielsweise die Vorrichtung gemäss der eingangs erwähnten CH-Patentschrift Nr. 669 401 verwendet werden.

Es besteht aber auch die Möglichkeit, die Nissenzahl, oder besser gesagt eine mit der effektiven Nissenzahl korrelierbare Grösse aus der für die Ermittlung der Faserbeeinträchtigung beschriebenen Frequenzanalyse zu gewinnen. Die Nissen sind die kleinsten und am intensivst reflektierenden Objekte, die sich durch das Gesichtsfeld des Sensors 11 (Figur 1) bewegen. Sie werden also im Diagramm der Figur 2 bei hohen Frequenzen und mit hohen Intensitäten erscheinen. Es kann also aus dem entsprechenden Frequenzspektrum ein weiteres Frequenzband FB.3 (in der Figur gestrichelt dargestellt) in einem Frequenzbereich höher als der Frequenzbereich FG der Garnitur ausgefiltert und seine Intensität gemessen werden. Die derart gewonnenen Werte können nach dem beschriebenen Verfahren weiterverarbeitet werden und liefern dann Werte, die einer Nissenzahl korreliert werden können oder aber, über entsprechende Eichwerte, die Nissenzahl selbst.

Aus dem Diagramm der Figur 2 ist im übrigen ersichtlich, dass im Zeitraum zwischen der Aufnahme des Frequenzspektrums A und der Aufnahme des Frequenzspektrums B die Nissenzahl zwar leicht abgenommen hat (weniger Intensität in FB.3), dass die Tambourgeschwindigkeit sich nicht verändert hat (gleiche Frequenz für das Maximum im möglichen Bereich für die Garniturfrequenz FQ), dass die längeren Fasern abgenommen (weniger Intensität in FB.2), die kürzeren aber zugenommen haben (mehr Intensität in FB.1). Aus dem relativen Verlauf der beiden Kurven A und B kann entnommen werden, dass die Kardierungsintensität, beispielsweise durch entsprechende Verstellung eines Kardenparameters (ausgenommen der Tambourgeschwindigkeit), zugenommen hat. In ähnlicher Weise würde das Personal die im einfachsten Fall angezeigten Ausgangswerte aus der Frequenzanalyse interpretieren.

Es kann sich als vorteilhaft erweisen, wenn die Messungen für die Faserbeeinträchtigung und für die Nissenzahl an verschiedenen Stellen in der Karde gemacht werden, sodass beispielsweise die die Fasern betreffenden Messungen auf dem Tambour, wo allein genügend kleine Faserdichten für eine solche Messung vorliegen, die die Nissen betreffenden Messungen aber auf der Abnehmerwalze, auf dem Abnehmer oder am offenen Vlies, beispielsweise mit einem Durchlichtverfahren, vorgenommen werden.

**Figur 4** zeigt nun eine beispielhafte Vorrichtung zur Durchführung des erfindungsgemässen Verfahrens. Über die Breite des Tambours sind beispielsweise vier optische Sensoren 11.1, 11.2, 11.3 und 11.4 in einer Reihe parallel zur Tambourachse verteilt. Die Gleichlichtquelle oder -quellen sind nahe der Sensorenreihe angebracht und in der Figur 4 nicht dargestellt. Die Sensoren sind vorteilhafterweise derart ausgestaltet, dass sie auf dem Vlies ein Rechteck "beobachten", das in Richtung der Vliesbreite ca. 20mm, in Richtung der Vliesbewegung ca. 0,5mm misst.

Da die Messung nicht einen exakten Wert eines physikalischen Qualitätsparameters ermitteln will, ist es nicht notwendig, dass die gesamte Breite des Vlieses abgetastet wird, das heisst, dass die gesamte Breite des Vlieses mit Sensoren versehen ist. Ebenfalls ist es nicht notwendig, dass die ganze Lange des Vlieses abgetastet wird, sodass die Signale der 4 Sensoren über einen langsamen und dadurch kostensparenden Multiplexer 40 auf einen einzigen Analysator geleitet werden können. Auf diese Weise werden auf 4 über die Breite verteilten örtlichen Teilbereichen zeitliche Samples der optischen Merkmale des Vlieses weiterverarbeitet.

Selbstverständlich ist es auch möglich, die ganze Breite des Vlieses mit Sensoren abzudecken und diese mit einem sehr schnellen Multiplexer abzufragen oder jeden Sensor mit einem eigenen Analysator zu versehen. Alle diese Ausführungsformen entsprechen aber nicht mehr dem letzten Punkt der Aufgabenstellung, der minimalen messtechnischen und elektronischen Aufwand fordert.

Die Verarbeitung der Messsignale der Sensoren können analog oder digital weiter verarbeitet werden, wodurch für die Auslegung der erfindungsgemässen Vorrichtung zwei Varianten entstehen.

## Patentansprüche

1. Verfahren zum Gewinnen von Messwerten zur Einstellung, Steuerung und/oder Regulierung der Kardenparameter, dadurch gekennzeichnet, dass aus den durch das Fasermaterial modulierten Ausgangssignalen von on-line durchgeführten, optischen Helligkeitsmessungen am Kardenvlies mittels einer elektronischen Answerteschaltung durch Frequenzanalyse Werte ermittelt werden, die mit der Kardierungsqualität, insbesondere mit der Faserbeeinträchtigung und/oder der Nissenzahl korreliert werden können wobei mittels eines Bandpassfilters ferner die in einem bestimmten, Frequenzbereich durch das Fasermaterial modulierten Ausgangssignale ausgefiltert werden, und dass diese ermittelten Werte als Kriterien für die Einstellung der Kardenparameter verwendet werden.

2. Verfahren nach Anspruch 1, **dadurch gekennzeichnet**, dass die durch die Frequenzanalyse ermittelten Werte die Intensitäten (I(FB)) in mindestens einem Frequenzband (FB) sind.

3. Verfahren nach Anspruch 2, **dadurch gekennzeichnet**, dass die Intensitäten (I(FB.1) und I(FB.2)) von zwei Frequenzbändern (FB.1 und FB.2), deren Frequenzbereich einem Bereich kleinerer und einem Bereich grösserer Faserlänge zugeordnet werden, ermittelt werden.

4. Verfahren nach Anspruch 3, **dadurch gekennzeichnet**, dass aus den beiden Intensitäten (I(FB.1) und I(FB.2)) ein Quotient gebildet wird.

5. Verfahren nach einem der Ansprüche 1 bis 4, **dadurch gekennzeichnet**, dass eine dritte Intensität (I(FB.3) in einem dritten Frequenzband (FB.3), dessen Frequenzbereich den Nissen zugeordnet wird, ermittelt wird.

6. Verfahren nach einem der Ansprüche 2 bis 5, **dadurch gekennzeichnet,** dass die Lage der Frequenzbänder im Frequenzspektrum entsprechend der Geschwindigkeit des ausgemessenen Vlieses reguliert wird.

7. Verfahren nach Anspruch 6, **dadurch gekennzeichnet,** dass zur Regulierung der Lage der Frequenzbänder im Frequenzspektrum eine Messung der Geschwindigkeit des Vlieses als Messgrösse verwendet wird.

8. Verfahren nach Anspruch 6, **dadurch gekennzeichnet,** dass die Frequenzbänder im Frequenzspektrum einen konstanten Abstand von derjenigen Frequenz (FG) haben, die im möglichen, der Garnitur zugeordneten Frequenzbereich die höchste Intensität aufweist.

9. Verfahren nach Anspruch 1, **dadurch gekennzeichnet,** dass die durch die Frequenzanalyse ermittelten Werte die Grenzfrequenzen von Frequenzbändern, die in empirisch ermittelten Frequenzbereichen liegen und deren Intensitäten einem empirisch ermittelten Wert entsprechen, sind.

10. Verfahren nach einem der Ansprüche 1 bis 9, **dadurch gekennzeichnet,** dass die optischen Messungen von örtlichen und zeitlichen Samples des Kardenvlieses gemacht werden.

11. Verfahren nach einem der Ansprüche 1 bis 10, **dadurch gekennzeichnet,** dass die durch die Frequenzanalyse ermittelten Werte örtlich und zeitlich gemittelt werden.

12. Verfahren nach einem der Ansprüche 1 bis 11, **dadurch gekennzeichnet,** dass die durch die Frequenzanalyse ermittelten Werte, entsprechende Mittelwerte oder aus solchen berechnete Grössen mit entsprechenden Eichwerten verglichen werden und dass aus dem Vergleich Grössen fuhr die Qualitätsmerkmale Faserbeeinträchtigung und Nissenzahl gewonnen werden.

13. Verfahren nach einem der Ansprüche 1 bis 12, **dadurch gekennzeichnet,** dass die durch die Frequenzanalyse ermittelten Werte oder aus solchen durch Verarbeitung entstandene Werte angezeigt werden.

14. Verfahren nach einem der Ansprüche 1 bis 13, **dadurch gekennzeichnet,** dass die durch die Frequenzanalyse ermittelten Werte oder aus solchen durch Verarbeitung entstandene Werte mit entsprechenden Grenzwerten verglichen werden und dass aus dem Vergleich Alarmsignale erstellt werden.

15. Verfahren nach einem der Ansprüche 1 bis 14, **dadurch gekennzeichnet,** dass die durch die Frequenzanalyse ermittelten Werte oder aus solchen durch Verarbeitung entstandene Werte zu Stellwerten für eine automatische Steuerung oder Regulierung der Karde verarbeitet werden.

16. Vorrichtung zum Gewinnen von Messwerten zur Einstellung, Steuerung und/oder Regulierung der Kardenparameter an einer Karde, die mindestens eine Lichtquelle (10) zum Beleuchten des Vlieses, mindestens einen optischen Sensor (11) zum Aufnehmen von vom Vlies reflektierenden Licht und eine elektronische Auswerteschaltung aufweist, dadurch gekennzeichnet, dass die elektronische Auswerteschaltung einen Analysator (12) umfasst, der im Betrieb Ausgangssignale des Sensors erhält, die on-line durchgeführten, optischen Helligkeitsmessungen am Kardenvlies entsprechen und einen Bandpassfilter aufweist, in welchem in einem bestimmten Frequenzbereich durch das Fasermaterial modulierte Ausgangssignale ausgefiltert werden und dass mittels Frequenzanalyse Werte ermittelt werden, die als Kriterien für die Einstellung der Karden parameter verwendet werden können.

## Claims

1. A method for obtaining measured values for setting, controlling and/or regulating the card parameters, characterized in that values are determined from output signals modulated by the fibre material from optical brightness measurements which are carried out on-line on the card fleece by means of an electronic evaluation circuit by frequency analysis, which values can be correlated with the carding quality, in particular with the fibre impairment and/or the nep count, and with the output signals which are modulated by the fibre material further being filtered out in a specific frequency band by means of a bandpass filter, and that said determined values are used as criteria for setting the card parameters.

2. A method as claimed in claim 1, characterized in that the values determined by the frequency analysis are the intensities (I(FB)) in at least one frequency band (FB).

3. A method as claimed in claim 2, characterized in that the intensities (I(FB.1) and I(FB.2)) of two frequency bands (FB.1 and FB.2) are determined whose frequency range is allocated to a range of smaller and a range of larger fibre length.

4. A method as claimed in claim 3, characterized in that a quotient is formed from the two intensities (I(FB.1) and I(FB.2)).

5. A method as claimed in one of the claims 1 to 4, characterized in that a third intensity (I(FB.3)) in a third frequency band (FB.3) is determined whose frequency range is allocated to the neps.

6. A method as claimed in one of the claims 2 to 5, characterized in that the position of the frequency bands in the frequency spectrum is regulated according to the speed of the measured fleece.

7. A method as claimed in claim 6, characterized in that for regulating the position of the frequency bands in the frequency spectrum a measurement of the speed of the fleece is used as measured variable.

8. A method as claimed in claim 6, characterized in that the frequency bands in the frequency spectrum have a constant distance from the frequency (FG) which is provided with the highest intensity in the possible frequency range allocated to the clothing.

9. A method as claimed in claim 1, characterized in that the values determined by the frequency analysis are the threshold frequencies of frequency bands which lie in empirically determined frequency ranges and whose intesities correspond to an empirically determined value.

10. A method as claimed in one of the claims 1 to 9, characterized in that the optical measurements are made from local and time samples of the card fleece.

11. A method as claimed in one of the claims 1 to 10, characterized in that the values determined by the frequency analysis are averaged locally and temporally.

12. A method as claimed in one of the claims 1 to 11, characterized in that the values determined by the frequency analysis, respective mean values or values calculated from the same are compared with respective calibration values and that values are gained from the comparison for the quality features of fibre impairment and nep count.

13. A method as claimed in one of the claims 1 to 12, characterized in that the values determined by frequency analysis or values arising from the same by processing are displayed.

14. A method as claimed in one of the claims 1 to 13, characterized in that the values determined by frequency analysis or values arising from the same by processing are compared with respective threshold values and that alarm signals are generated from the comparison.

15. A method as claimed in one of the claims 1 to 14, characterized in that the values determined by frequency analysis or values arising from the same by processing are processed into a manipulated variable for an automatic control unit or for regulating the card.

16. An apparatus for obtaining measured values for setting, controlling and/or regulating the card parameters in a card, which is provided with at least one light source (10) for lighting the fleece, at least one optical sensor (11) for receiving light reflected from the fleece and an electronic evaluation circuit, characterized in that the electronic evaluation circuit comprises an analyzer (12) which during operation receives output signals from the sensor which correspond to optical brightness measurements carried out on-line and which comprises a bandpass filter in which in a certain frequency range the output signals which are modulated by the fibre material are filtered out, and that by means of frequency analysis values are determined which can be used as criteria for the setting of the card parameters.

## Revendications

1. Procédé utilisé pour l'exploitation de valeurs de mesure servant au réglage, à la commande et/ou à la régulation des paramètres de carde,
caractérisé par le fait que,
à partir des signaux de sortie, modulés par la matière fibreuse et provenant de mesures optiques de luminosité faites sur le voile de carde et effectuées en ligne, des valeurs sont déterminées par analyse de fréquences, à l'aide d'une commutation électronique d'interprétation, qui peuvent être mises en corrélation avec la qualité du cardage, particulièrement avec l'influence néfaste sur les fibres et/ou le nombre de boutons, et où, dans une zone de fréquences déterminée, les signaux de sortie modulés par la matière fibreuse, sont en outre filtrés à l'aide d'un passe-bande, et que ces valeurs déterminées sont utilisées comme critères pour le réglage des paramètres de carde.

2. Procédé selon revendication 1,
caractérisé par le fait que
les valeurs déterminées par analyse de fréquences sont les intensités (I(FB)) situées dans au moins une bande de fréquences (FB).

3. Procédé selon revendication 2,
caractérisé par le fait que
les intensités (I(FB.1) et I(FB.2)) de deux bandes de fréquences (FB.1 et FB.2) sont déterminées, bandes de fréquences dont les zones de fréquences sont attribuées à une zone de longueur de fibres plus petite et à une zone de longueur de fibres plus grande.

4. Procédé selon revendication 3,
caractérisé par le fait
qu'un quotient est formé à partir des deux intensités (I(FB.1) et I(FB.2)).

5. Procédé selon une des revendications 1 à 4,
caractérisé par le fait
qu'une troisième intensité (I(FB.3)) est déterminée dans une troisième bande de fréquences (FB.3), dont la zone de fréquences est attribuée aux boutons.

6. Procédé selon une des revendications 2 à 5,
caractérisé par le fait que
la position des bandes de fréquences dans le spectre de fréquences est réglée en fonction de la vitesse du voile mesuré.

7. Procédé selon revendication 6,
caractérisé par le fait que,
pour le réglage de la position des bandes de fréquences dans le spectre de fréquences, une mesure de la vitesse du voile est utilisée comme variable mesurée.

8. Procédé selon revendication 6,
caractérisé par le fait que,
dans le spectre de fréquences, les bandes de fréquences possèdent une distance constante par rapport à la fréquence (FG) qui présente l'intensité la plus élevée, dans la zone de fréquences possible attribuée à la garniture.

9. Procédé selon revendication 1,
caractérisé par le fait que
les valeurs déterminées par l'analyse de fréquences sont les fréquences limites des bandes de fréquences qui sont situées dans des zones de fréquences déterminées empiriquement, et dont les intensités correspondent à une valeur déterminée empiriquement.

10. Procédé selon une des revendications 1 à 9,
caractérisé par le fait que
les mesures optiques sont effectuées sur des échantillons locaux et temporaires du voile de carde.

11. Procédé selon une des revendications 1 à 10,
caractérisé par le fait que
les valeurs déterminées par l'analyse de fréquences sont moyennées localement et temporairement.

12. Procédé selon une des revendications 1 à 11,
caractérisé par le fait que
les valeurs déterminées par l'analyse de fréquences, des valeurs moyennes correspondantes, ou des grandeurs calculées à partir de celles-ci, sont comparées avec des valeurs étalon correspondantes, et que, à partir de ces comparaisons, des grandeurs sont exploitées pour déterminer les caractéristiques de qualité, l'influence néfaste sur les fibres et le nombre de boutons.

13. Procédé selon une des revendications 1 à 12,
caractérisé par le fait que
les valeurs déterminées par l'analyse de fréquences ou des valeurs obtenues par traitement de celles-ci, sont visualisées.

14. Procédé selon une des revendications 1 à 13,
caractérisé par le fait que
les valeurs déterminées par l'analyse de fréquences ou des valeurs obtenues par traitement de celles-ci, sont comparées avec des valeurs limites correspondantes, et que des signaux d'alarme sont réalisés à partir de la comparaison.

15. Procédé selon une des revendications 1 à 14,
caractérisé par le fait que
les valeurs déterminées par l'analyse de fréquences ou des valeurs obtenues par traitement de celles-ci, sont converties en valeurs de réglage utilisées pour un asservissement automatique ou une régulation de la carde.

16. Dispositif utilisé pour l'exploitation de valeurs de mesure servant au réglage, à la commande et/ou à la régulation des paramètres de carde dans une carde, lequel possède au moins une source de lumière (10) pour illuminer le voile, au moins un détecteur optique (11) servant à la réception de la lumière réfléchissante du voile, et une commutation électronique d'interprétation,
caractérisé par le fait que
la commutation électronique d'interprétation comprend, un analyseur (12) qui, pendant la marche, reçoit des signaux de sortie du détecteur, lesquels correspondent à des mesures optiques de luminosité faites sur le voile de carde et effectuées en ligne, et un passe-bande dans lequel des signaux de sortie, modulés par la matière fibreuse, sont filtrés dans une zone de fréquences déterminée, et que des valeurs, déterminées à l'aide d'analyse de fréquences, peuvent être utilisées comme critères pour le réglage des paramètres de carde.
